# EUROPÄISCHE PATENTANMELDUNG

(11) **EP 1 090 613 A1**
(43) Veröffentlichungstag der Anmeldung: **11.04.2001**
(21) Anmeldenummer: 00121892.4
(22) Anmeldetag: 06.10.2000
(51) Int. Cl.: A61F 5/44

(54) **Medizinisches Gefäss und Schutzhülle für ein medizinisches Gefäss**

(30) Priorität: 06.10.1999 DE 19948018
(71) Anmelder: Mohr, Gabriele, 50127 Bergheim (DE)
(72) Erfinder: Mohr, Gabriele, 50127 Bergheim (DE)
(74) Vertreter: Castell, Klaus, Dr.-Ing.

(57) **Zusammenfassung**

Ein medizinisches Gefäß zur Aufnahme von Flüssigkeiten aus dem menschlichen Körper wird erfindungsgemäß so ausgebildet, dass eine im Aufnahmekörper befindliche Flüssigkeit nicht oder nur an bestimmten Stellen von außen sichtbar ist. Eine Weiterbildung der Erfindung sieht vor, dass das medizinische Gefäß eine Schutzhülle aufweist, die derart über den Aufnahmekörper schieb-, zieh-, oder steckbar ist, dass eine im Aufnahmekörper befindliche Flüssigkeit nicht oder nur an bestimmten Stellen von außen sichtbar ist. Vorteilhaft ist es, wenn das medizinische Gefäß eine Befestigungseinrichtung zur Befestigung an einem Bettrahmen und zur Verbindung mit anderen medizinischen Gefäßen aufweist.

Diese Ausgestaltungen führen dazu, die psychologische Akzeptanz der jeweiligen Gefäße bei dem Patienten zu erhöhen, so dass die Patienten sich bei er Anwendung der jeweiligen Gefäße wohler fühlen.

## Beschreibung

Die Erfindung betrifft ein medizinisches Gefäß zur Aufnahme von Flüssigkeiten aus dem menschlichen Körper, wie Spritze, Urin- und Stomabeutel, das heißt Beutel für künstliche Darmausgänge, oder Beutel zur Infusion und Injektion von Flüssigkeiten in den menschlichen Körper, wie Spritze, Infusionsbeutel und -flasche, wobei das Gefäß einen Aufnahmekörper für die Flüssigkeit aufweist. Die Erfindung betrifft ferner eine Schutzhülle für solche Gefäße.

Solche Gefäße sind in unterschiedlichster Form und aus unterschiedlichen, jedoch stets durchsichtigen Materialien, nämlich Kunststoff und Glas, bekannt und haben sich in der Praxis durchaus bewährt.

Allerdings besteht bei den bekannten Gefäßen das Problem der psychologischen Akzeptanz bei den Patienten, die es oftmals als unangenehm empfinden, zu sehen, wie sich z. B. eine Spritze mit ihrem Blut oder über einen entsprechenden Katheter ein Urinbeutel mit ihrem Urin füllt. Dieser Nachteil wurde bislang vom medizinischen Personal billigend in Kauf genommen, da dem genannten Nachteil der Vorteil gegenübersteht, daß das Personal so mit einem Blick den Befüllungsgrad des jeweiligen Gefäßes erkennen kann.

Davon ausgehend liegt der Erfindung die Aufgabe zugrunde, ein medizinisches Gefäß und eine Schutzhülle für ein solches Gefäß anzugeben, welche dem Patienten den Anblick auf seine Körperflüssigkeiten oder einer in seinen Körper einzuführende Flüssigkeit ersparen, ohne jedoch die Arbeit des medizinischen Personals dadurch zu erschweren.

Die Aufgabe wird gelöst von einem Gefäß der eingangs genannten Art, bei welchem der Aufnahmekörper derart ausgebildet ist, daß eine im Aufnahmekörper befindliche Flüssigkeit nicht oder nur an bestimmten Stellen von außen sichtbar ist.

Es hat sich nämlich gezeigt, daß in vielen Fällen eine exakte Kontrolle des Befüllungsgrades von außen gar nicht notwendig ist, da er sich z.B. bei Urin- oder Stomabeuteln an der Form ablesen läßt und sich, z.B. bei Infusionsspritzen, die aus Einmalampullen aufgezogen werden, wobei der gesamte Inhalt der Einmalampulle dem Patienten intravenös gegeben werden soll, bei vollständiger Entleerung der Ampulle von selbst ergibt.

Die Erfindung hat den großen Vorteil, daß die psychologische Akzeptanz der jeweiligen Gefäße bei den Patienten wesentlich erhöht wird, die Patienten sich also bei der Anwendung der jeweiligen Gefäße wohler fühlen, wobei eine Therapie oder Behandlung erwiesenermaßen um so erfolgreicher ist, um so mehr der Patient alle im Rahmen der Behandlung oder Therapie erforderlichen Maßnahmen - also auch die Verwendung von Gefäßen der hier in Frage stehenden Art - akzeptiert. Die Erfindung stellt also einen Baustein bei der Verbesserung der patientengerechten also einen Baustein bei der Verbesserung der patientengerechten Versorgung dar.

Bei einer bevorzugten Ausführungsform weist das Gefäß wenigstens ein Sichtfenster oder einen Sichtstreifen auf, welches/welcher vorteilhaft die Kontrolle des Flüssigkeitsstandes im Aufnahmekörper erlaubt. Dazu kann neben dem Sichtfenster bzw. Sichtstreifen eine Skala zur Bestimmung des Füllstandes aufgebracht werden.

Insbesondere bei Gefäßen, die zur Aufnahme geruchsintensiver Substanzen wie Urin vorgesehen sind, kann die Gefäßinnenseite vorteilhaft zumindest partiell mit einer geruchsbindenden oder geruchshemmenden Substanz, insbesondere einem ätherische Öl oder Natrium-Kupfer-Chlorophyllin, versehen werden. Weist das Gefäß wie z.B. manche Stomabeutel einen Filter, z.B. einen Aktivkohlefilter auf, so kann auch der Filter zumindest partiell mit einer geruchsbindenden oder geruchshemmenden Substanz, insbesondere einem ätherische Öl oder Natrium-Kupfer-Chlorophyllin, versehen sein. Besonders vorteilhaft für die Geruchshemmung bei gattungsgemäßen Gefäßen ist auch Bismuthsalizylat. Diese Substanz kann Hydrogensulfid wirksam binden, welches mit Methangas hauptverantwortlich für den unangenehmen Geruch ist.

Alternativ oder zusätzlich kann die Gefäßinnenseite vorteilhaft zumindest partiell mit einer bakterien- und/oder keimtötenden und/oder das Ballooning (worunter das Phänomen verstanden wird, daß sich elastische Gefäße wie Stomabeutel z.B. aufgrund von bei Gährungsprozessen austretenden Gase aufblähen) hemmenden Substanzen, wie insbesondere Siliciumdioxid, versehen werden.

In besonders vorteilhafter Weiterbildung der Erfindung ist wenigstens ein von der Gefäßaußenseite ablesbarer mit einer in das Gefäß eingebrachten Substanz reagierender Teststreifen zum Nachweis wenigstens eines bestimmten in der Substanz eventuell vorhandenen Stoffes vorgesehen. Solche Teststreifen können beispielsweise sein: Teststreifen zum Nachweis von Blut im Urin, Teststreifen zum Nachweis von Eiweiß im Urin, Teststreifen zum Nachweis von Nitrat im Urin, Teststreifen zum Nachweis von Glukose im Urin, Teststreifen zum Bestimmen des Urin-pH-Wertes, Teststreifen zum Bestimmen bestimmter Blutwerte, etc. Diese Teststreifen ermöglichen vorteilhaft eine sofortige Kontrolle ausgeschiedener oder entnommener Körperflüssigkeiten auf bestimmte Anomalien, so daß Gefahren frühzeitig erkannt werden können. Durch entsprechende Ausbildung der Teststreifen ist es dabei vorteilhaft möglich, daß die Patienten selbst erkennen können, ob bestimmte Gefahren vorliegen oder ob sich die Anteile der nachgewiesenen Substanzen im normalen Bereich bewegen. Dies kann zum Beispiel durch entsprechende Farbgebung bei Nachweis bestimmter Mengen des jeweils betrachteten Stoffes geschehen (z.B. Nachweis von Blut im Urin bei Patienten mit erhöhter Gefahr von bakteriellen Harnwegsinfektionen: zeigt der Teststreifen nach Ausscheidung von Urin grün, ist kein Blut im Urin enthalten, verfärbt sich der Teststreifen dagegen rot, sollte schnell ein Arzt konsultiert werden, da Blut im Urin nachgewiesen wurde). Alternativ oder zusätzlich kann neben dem Teststreifen auch eine Vergleichsskala zum Ablesen des Teststreifens aufgebracht werden.

Die Teststreifen werden vorteilhafter Weise an der Innenwandung des Gefäßes befestigt, so dass sie mit dem Gefäßinhalt in Berührung kommen. Eine vorteilhafte Ausführungsvariante sieht vor, dass die Gefäßwandung an der Stelle der Teststreifen durchsichtig ist, so dass die Färbung der Streifen von außen sichtbar ist, obwohl sich die Teststreifen im Gefäß befinden.

Alternativ oder zusätzlich zu den genannten Teststreifen kann Insbesondere bei Gefäßen wie Stomabeutel in dem Gefäß wenigstens ein herausnehmbarer mit einer in das Gefäß eingebrachten Substanz reagierender Teststreifen zum Nachweis wenigstens eines bestimmten in der Substanz eventuell vorhandenen Stoffes vorgesehen werden. Ein solcher Teststreifen kann z.B. zum Nachweis von Blut im Stuhl (Benzidinprobe) ausgebildet sein.

Bei einer vorteilhaften Ausführungsform der vorgenannten Gefäße ist die chemische Zusammensetzung der Gefäße in von außen ablesbarer Form an den Gefäßen angegeben, so daß Allergiker leicht erkennen können, ob ein Gefäß für sie geeignet ist oder nicht.

Die Aufgabe wird zum anderen gelöst von einer Schutzhülle für Gefäße der eingangs genannten Art, wobei die Schutzhülle so ausgebildet und derart über den Aufnahmekörper des jeweiligen medizinischen Gefäßes schieb-, zieh- oder steckbar ist, daß eine im Aufnahmekörper befindliche Flüssigkeit nicht oder nur an bestimmten Stellen von außen sichtbar ist. Eine solche Schutzhülle hat nicht nur die bereits genannten Vorteile, sondern erlaubt auch die Weiterverwendung bereits vorhandener durchsichtiger Gefäße, über die dann bei Bedarf nur die Schutzhülle gestülpt werden muß.

Gefäß bzw. Schutzhülle können vorteilhaft direkt aus opakem Material, z. B. einem entsprechend eingefärbten Kunststoff hergestellt sein. Es ist aber auch möglich, Gefäß bzw. Schutzhülle zumindest partiell mit einem opaken Stoff zu beschichten, also z. B. mittels dem jeweiligen Gegenstand angepaßter Verfahren mit einer geeigneten opaken Farbe zu bedrucken. Außerdem ist an Stelle der üblichen Hautfarben eine bunte Oberfläche vorteilhaft, die einfarbig oder vielfarbig in ansprechendem Design ausgeführt sein kann.

Damit können vorteilhaft auch bereits vorhandene Gefäße in der gewünschten Weise nachgerüstet werden. Sowohl beim Gefäß als auch bei der Schutzhülle sind sowohl einfarbige flächige Beschichtungen als auch Motivdrucke möglich. So ist es z. B. möglich, eine Spritze zum Abnehmen von Blut bei Kindern mit freundlichen Motiven, z. B. Blumen oder Tieren, zu bedrucken und dabei einen Sichtstreifen oder auch entlang des Aufnahmekörpers an verschiedenen Stellen verteilte Sichtfenster freizulassen, die die Kontrolle des Flüssigkeitsstandes im Aufnahmekörper der Spritze erlauben.

Solche Sichtfenster oder Sichtstreifen zur Kontrolle des Flüssigkeitsstandes im jeweiligen Aufnahmekörper können sowohl bei den Gefäßen als auch bei entsprechenden Schutzhüllen vorgesehen sein. In vielen Anwendungsfeldern kann jedoch - wie erwähnt - auf solche Sichtstreifen oder Sichtfenster gänzlich verzichtet werden.

Besonders bei Gefäßen wie z. B. Urinbeuteln, die in direktem Kontakt mit einem Patienten kommen, kann in zweckmäßiger Weiterbildung der Erfindung das medizinische Gefäß zumindest partiell mit einer veloursartigen Oberfläche, gelegentlich als Vließoberfläche bezeichnet, versehen sein, was zu einer angenehmeren Haptik dieser Gefäße führt.

Im Rahmen des Erfindungsgedankens sind zahlreiche Abwandlungen und Weiterbildungen möglich, die sich z.B. auf die Zahl und Ausgestaltung der Sichtfenster und die Ausbildung der Schutzhülle beziehen. So kann die Schutzhülle z.B. aus festem Material hergestellt und damit nicht nur den Blick auf das Aufnahmegefäß in der gewünschten Weise verhindern, sondern gleichzeitig das jeweillige Gefäß auch besonders gut vor Beschädigungen schützen.

Die der Erfindung zur Grunde liegende Aufgabe, ein medizinisches Gefäß anzugeben, welches dem Patienten den Anblick auf seine Körperflüssigkeiten erspart, ohne jedoch die Arbeit des medizinischen Personals dadurch zu erschweren, wird auch mit einem medizinischen Gefäß gelöst, das eine Befestigungseinrichtung zur Befestigung an einem Bettrahmen und zur Verbindung mit anderen medizinischen Gefäßen aufweist.

Diese Befestigungseinrichtung erlaubt es, mehrere medizinische Gefäße gleichzeitig am Bettrahmen zu befestigen, um somit die Vielzahl benötigter medizinischer Gefäße außerhalb des Sichtbereiches des Patienten anzuordnen. Auch durch diese Anordnung bleibt dem Patienten der Blick auf die medizinischen Geräte erspart.

Bekannte Befestigungseinrichtungen sind nur zur Befestigung eines einzelnen medizinischen Gefäßes geeignet und daher für die Befestigung mehrerer medizinischer Gefäße am Bettrahmen nicht geeignet. Dies führt dazu, daß bisher im Krankenzimmer an verschiedensten Stellen unterschiedliche medizinische Gefäße aufgestellt wurden. Diese Gefäße dienten einerseits dazu, Körperflüssigkeiten, die aus dem Körper entnommen wurden, aufzufangen oder dem Körper zuzuführende Flüssigkeiten aufzunehmen.

Die erfindungsgemäße Befestigungseinrichtung ermöglicht es erstmals, sämtliche medizinischen Gefäße nebeneinander ordentlich am Bettgestell zu befestigen. Durch die nahe Anbindung der Gefäße ans Bett können relativ kurze Schläuche verwendet werden und die Gefahr einer Beschädigung der Schläuche oder eines Ziehens an einem Schlauch wird deutlich verringert.

Die definierte Anordnung der Gefäße relativ zum Bettgestell ermöglicht es, die Gefäße an einer dem Patienten abgewandten Seite mit einer Befüllungsanzeige zu versehen. Bei einer undurchsichtigen Ausführungsform der Gefäße kann beispielsweise an der dem Patienten abgewandten Seite ein senkrecht verlaufenden durchsichtigen Bereich vorgesehen sein, um dem medizinischen Personal das Ablesen des Befüllungsgrades zu erleichtern. Da sämtliche Gefäße nebeneinander angeordnet sind, kann das medizinische Personal auf einen Blick den Füllungsgrad der Gefäße erfassen und die notwendigen Maßnahmen ergreifen.

Eine bevorzugte Ausführungsvariante sieht vor, dass die Befestigungseinrichtung einen im wesentlichen U-förmigen Träger aufweist, der auf den Bettrahmen aufsteckbar ist. Eine derartige U-Schiene ist einfach herstellbar. Diese U-Schiene kann an ihrer Innenseite eine weiche und vorzugsweise elastische Auflage aufweisen. Dies erlaubt es, die U-Schiene mit ihrer Öffnung nach unten auf einen Vierkantträger eines Bettgestells aufzustecken. Die weiche oder elastische Auflage, die auch punktuell als Polster oder Streifenelement ausgebildet sein kann, verhindert ein Verkratzen des Bettgestellträgers und erlaubt ein sicheres - nicht klapperndes - Anbringen des medizinischen Gefäßes am Bettrahmen.

Eine Weiterbildung der Erfindung sieht vor, daß die Befestigungseinrichtung ein Stecksystem, vorzugsweise nach dem Nut- und Federprinzip, aufweist. Die Befestigung des medizinischen Gefäßes am Bettrahmen wird somit leicht lösbar verwirklicht, so daß das medizinische Personal schnell und einfach die medizinischen Gefäße wie beispielsweise Redonflaschen auswechseln kann.

Vorteilhaft ist es, wenn die Befestigungseinrichtung ein Clips- oder Rastsystem aufweist. Das Einrasten in einer bestimmten Position sichert die genaue Lage des medizinischen Gefäßes relativ zum U-förmigen Träger und das medizinische Personal fühlt bei der Befestigung des Gefäßes am Träger, daß das Gefäß sicher an der U-Schiene eingerastet ist.

Hierzu wird vorgeschlagen, am Gefäß einen Steckkopf und am Bettrahmen eine Steckkopfaufnahme anzuordnen. Dies erlaubt es, auf einfache Art und Weise das Gefäß an einer am Bettrahmen befestigten Halterung anzustecken.

Aus statischen Gründen ist es vorteilhaft, wenn der Steckkopf einstückig mit dem Gefäß ausgebildet ist. Da die Gefäße meistens aus Kunststoff oder Glas hergestellt sind, wird somit vorgeschlagen, am Gefäß einen Nippel anzuformen, der eine Steckverbindung mit einer am Bettgestell angeordneten Steckkopfaufnahme ermöglicht.

Um auch herkömmliche Gefäße mittels der Befestigungseinrichtung am Bettrahmen zu fixieren, wird weiterhin vorgeschlagen, dass der Steckkopf mittels eines vorzugsweise elastischen Bandes am Gefäß befestigt ist.

Eine einfache Befestigung des Bandes am Gefäß wird dadurch erreicht, daß am Band ein Klettverschluss vorgesehen wird, der es erlaubt, das Band um das medizinische Gefäß herumzulegen und durch einfaches Aufeinanderlegen des Bandendes auf den Bandrücken eine sichere Befestigung zu erzielen.

Eine einfache Ausbildung der Steckkopfaufnahme sieht vor, dass hierzu ein Blech mit Löchern vorgesehen wird. Dieses Blech ist derart beabstandet zum Bettrahmen anzuordnen, dass ein Steckkopf in eines der Löcher eingesteckt werden kann. Eine einfache Ausführungsvariante sieht vor, dass das Blech eine Lochreihe aufweist. Vorteilhaft ist jedoch die Verwendung eines Lochbleches mit einer Vielzahl an Löchern, das es erlaubt, unterschiedliche Flaschen in verschiedenen Positionen ggf. auch mit mehreren Steckköpfen am U-Profilträger zu befestigen.

Ein bevorzugtes Ausführungsbeispiel ist in der Zeichnung dargestellt und wird im folgenden näher erläutert.

Es zeigen:
- Figur 1: eine perspektivische Ansicht zweier an einem Bettrahmen befestigter medizinischer Gefäße,
- Figur 2: einen Schnitt durch ein medizinisches Gefäß mit Befestigungseinrichtung und
- Figur 3: schematisch ein medizinisches Gefäß mit Teststreifen.

Die Befestigungseinrichtung 3 ist ein U-förmiger Träger, der aus einem Lochprofil gebogen ist. Die Löcher sind in einem regelmäßigen Lochraster angeordnet und aus herstellungstechnischen Gründen über die gesamte U-Schiene verteilt, obwohl nur im vorderen Bereich der U-Schiene zur Befestigung der medizinischen Gefäße Löcher notwendig sind.

An der Innenseite der U-förmigen Schiene sind Abstandshalter 5, 6 mit Enden 7, 8 aus einem nicht kratzenden Material und gegenüberliegend Polster 9, 10 aus ebenfalls nicht kratzendem Material vorgesehen, um die U-Schiene 3 auf das Bettgestell 4 aufzustecken zu können, ohne dabei den Lack des Gestelles zu beschädigen.

An den medizinischen Gefäßen 1 und 2, die im vorliegenden Fall als Redonflaschen ausgebildet sind, ist jeweils mit einem Band 11, 12 ein Nippel 13 befestigt. Dieser Nippel 13 ist so ausgebildet, daß er in ein Loch 14 des Lochblechs 3 einsteckbar ist, um die Flasche 11 bzw. 12 fest am U-förmigen Träger zu halten. Im vorliegenden Ausführungsbeispiel ist je Flasche nur ein befestigter Nippel dargestellt. Verständlicherweise können auch mehrere Nippel und Bänder an einer Flasche befestigt werden.

Die Bänder 11 und 12 sind elastisch ausgebildet, und weisen auf ihrer Vorder- und Rückseite gegensätzliche Elemente einer Klettverbindung auf, so daß durch einfaches Umschlingen der Flasche mittels eines der Bänder 11, 12 der Nippel 13 fest an dem medizinischen Gefäß befestigt werden kann.

Im vorliegenden Fall ist am medizinischen Gefäß das männliche Teil der Steckverbindung vorgesehen, während das weibliche Teil von dem Trägersystem gebildet wird. Selbstverständlich kann auch am Trägersystem der männliche Teil befestigt werden und die Flasche den weiblichen Teil aufweisen.

Das beschriebene Trägersystem eignet sich besonders für medizinische Gefäße. Es können jedoch auch Untersuchungsgeräte wie Meß- und Diagnoseeinheiten am Trägersystem befestigt werden. In diesem Fall ist das medizinische Gerät nicht wie die vorliegenden Flaschen 1 und 2 mittels eines Schlauches 15, 16 sondern mittels eines Kabels oder auch kabellos mit dem Patienten verbunden.

Im Ausführungsbeispiel ist der Nippel 13 mittels eines Bandes an den Flaschen 1 bzw. 2 befestigt. Vorteilhaft ist es, wenn der Nippel direkt an der Flasche angeformt ist, da sich dann die Befestigung des Nippels an der Flasche erübrigt.

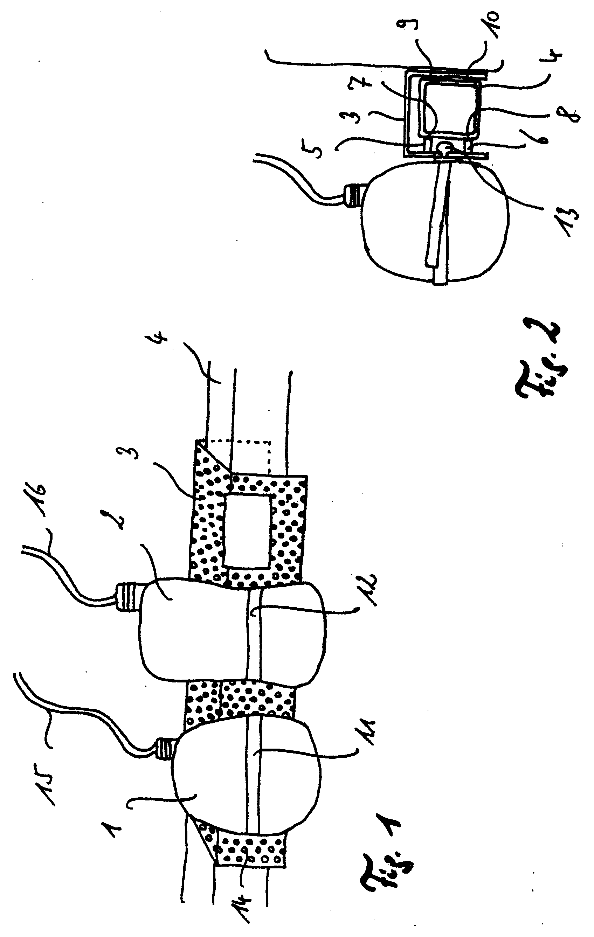

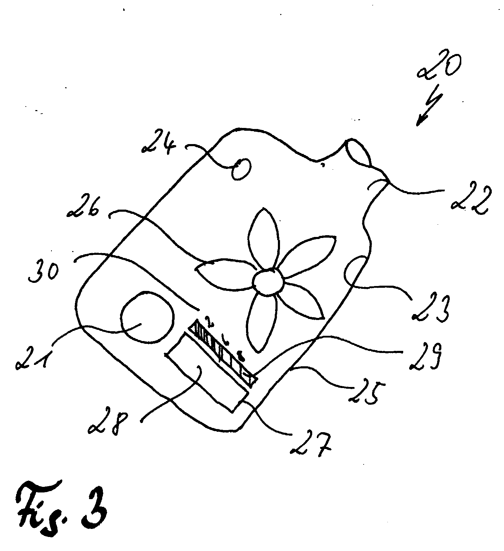

Der in Figur 3 gezeigte Stomabeutel 20 hat einen Eingang 21 und einen Ausgang 22. Die Beutelinnenseite 23 ist mit Bismuthsubsalizylat beschichtet und ein Geruchsfilter 24 ist ebenfalls mit dieser Substanz versehen, um die Entwicklung von Gerüchen schon im Beutel zu hemmen und letztlich im Filter 24 für eine Geruchsreduktion zu sorgen.

Die Beutelaußenseite 25 ist mit einer Farbschicht versehen, auf die bunte Motive 26 aufgedruckt sind. Der Beutel ist dadurch undurchsichtig bis auf das Sichtfenster 27, hinter dem ein Teststreifen 28 angeordnet ist. Der Teststreifen 28 befindet sich im Beutel 20. Der Streifen 28 hat einen Oberflächenbereich, der sich entsprechend dem Beutelinhalt verfärbt. Dieser Oberflächenbereich ist an der Innenseite 23 des Beutels 20 im Bereich des Fensters 27 angeordnet, so dass die Verfärbung durch das Fenster 27 von außen wahrgenommen werden kann.

Neben dem Fenster 27 ist ein Vergleichsstreifen 29 als Farbverlauf eingefärbt und daneben ist wiederum eine Werteskala angebracht. Dies erlaubt es, die Farbe des Teststreifens 28 im Fenster 27 wahrzunehmen und die entsprechende Position am Farbverlauf mit der gleichen Einfärbung zu ermitteln. Anschließend kann die in der Höhe dieser Position angegebene Ziffer der Skala 30 abgelesen werden, um einen Zahlenwert zu erhalten, der der Einfärbung des Teststreifens 28 entspricht.

## Patentansprüche

1. Medizinisches Gefäß zur Aufnahme von Flüssigkeiten aus dem menschlichen Körper, wie Spritze, Urin- und Stomabeutel (Beutel für künstliche Darmausgänge), oder Beutel zur Infusion und Injektion von Flüssigkeiten in den menschlichen Körper, wie Spritze, Infusionsbeutel und -flasche, wobei das Gefäß einen Aufnahmekörper für die Flüssigkeit aufweist, dadurch gekennzeichnet, daß der Aufnahmekörper derart ausgebildet ist, daß eine im Aufnahmekörper befindliche Flüssigkeit nicht oder nur an bestimmten Stellen von außen sichtbar ist.

2. Gefäß nach Anspruch 1, dadurch gekennzeichnet, daß es mit einer bunten Oberfläche oder aus opakem Material hergestellt ist.

3. Gefäß nach Anspruch 1 oder 2, dadurch gekennzeichnet, daß es zumindest partiell mit einem opaken Stoff beschichtet ist.

4. Gefäß nach einem der Ansprüche 1 bis 3, dadurch gekennzeichnet, daß es zumindest partiell mit einer veloursartigen Oberfläche versehen ist.

5. Gefäß nach einem der Ansprüche 1 bis 4, dadurch gekennzeichnet, daß wenigstens ein Sichtfenster oder ein Sichtstreifen vorgesehen ist, welches/welcher die Kontrolle des Flüssigkeitsstandes im Aufnahmekörper erlaubt.

6. Gefäß nach einem der Anspruch 5, dadurch gekennzeichnet, daß neben dem Sichtfenster bzw. Sichtstreifen eine Skala zur Bestimmung des Füllstandes aufgebracht ist.

7. Gefäß nach einem der Ansprüche 1 bis 6, dadurch gekennzeichnet, daß die Gefäßinnenseite zumindest partiell mit einer geruchsbindenden oder geruchshemmenden Substanz, insbesondere einem ätherische Öl oder Natrium-Kupfer-Chlorophyllin oder Bismuthsubsalizylat, versehen ist.

8. Gefäß nach einem der Ansprüche 1 bis 7, dadurch gekennzeichnet, daß die Gefäßinnenseite zumindest partiell mit einer bakterien-und/oder keimtötenden und/oder das Ballooning hemmenden Substanz, insbesondere Siliciumdioxid, versehen ist.

9. Gefäß nach einem der Ansprüche 1 bis 8, dadurch gekennzeichnet, daß wenigstens ein von der Gefäßaußenseite ablesbarer mit einer in das Gefäß eingebrachten Substanz reagierender Teststreifen zum Nachweis wenigstens eines bestimmten in der Substanz eventuell vorhandenen Stoffes vorgesehen ist.

10. Gefäß nach Anspruch 9, dadurch gekennzeichnet, daß neben dem Teststreifen eine Vergleichsskala zum Ablesen des Teststreifens aufgebracht ist.

11. Gefäß nach einem der Ansprüche 1 bis 10, dadurch gekennzeichnet, daß in dem Gefäß ein herausnehmbarer mit einer in das Gefäß eingebrachten Substanz reagierender Teststreifen zum Nachweis wenigstens eines bestimmten in der Substanz eventuell vorhandenen Stoffes vorgesehen ist.

12. Gefäß nach einem der Ansprüche 1 bis 11, dadurch gekennzeichnet, daß die chemische Zusammensetzung des Gefäßes in von außen ablesbarer Form an dem Gefäß angegeben ist.

13. Gefäß nach einem der Ansprüche 1 bis 12 mit einem Filter, insbesondere einem Aktivkohlefilter, dadurch gekennzeichnet, daß der Filter zumindest partiell mit einer geruchsbindenden oder geruchshemmenden Substanz, insbesondere einem ätherische Öl oder Natrium-Kupfer-Chlorophyllin oder Bismuthsubsalizylat, versehen ist.
